# EUROPÄISCHE PATENTSCHRIFT

(11) **EP 1 054 623 B1**
(45) Veröffentlichungstag und Bekanntmachung des Hinweises auf die Patenterteilung: **26.10.2005**
(21) Anmeldenummer: 99910132.2
(22) Anmeldetag: 11.02.1999
(51) Int. Cl.: A61B 6/08, A61N 5/10

(54) **REPRODUZIERBARE POSITIONS- ODER HALTUNGSERKENNUNG VON VERFORMBAREN KÖRPERN**
REPRODUCIBLE POSITION OR LOCATION IDENTIFICATION OF DEFORMABLE BODIES
IDENTIFICATION REPRODUCTIBLE DE POSITION OU DE TENUE DE CORPS DEFORMABLES

(30) Priorität: 13.02.1998 DE 19805917
(43) Veröffentlichungstag der Anmeldung: 29.11.2000
(73) Patentinhaber: Müller, Reinhold G., 91080 Marloffstein (DE); Klöck, Stephan, 8280 Kreuzlingen (CH)
(72) Erfinder: Müller, Reinhold G., 91080 Marloffstein (DE); Klöck, Stephan, 8280 Kreuzlingen (CH)
(74) Vertreter: Hafner, Dieter
(86) Internationale Anmeldenummer: PCT/DE1999/000400
(87) Internationale Veröffentlichungsnummer: WO 1999/040846

(56) Entgegenhaltungen:
- EP-A- 0 560 331
- DE-A- 3 436 444
- DE-A- 3 508 730
- US-A- 4 365 341
- US-A- 5 446 548

## Beschreibung

Die Erfindung betrifft ein Verfahren zur reproduzierbaren Positions- oder Haltungserkennung oder Lagerung von dreidimensionalen, beweglichen und verformbaren Körpern oder Körperteilen bezogen auf eine Unterlage und/oder einen über dieser Unterlage definierten Raumpunkt oder ein Raumkoordinatensystem.

Insbesondere ist ein Verfahren angesprochen, das an lebenden Körpern, insbesondere auch an lebenden menschlichen Patientenkörpern, durchgeführt werden kann.

### Stand der Technik:

In unterschiedlichen technischen und medizinisch-technischen Anwendungsbereichen besteht das Bedürfnis, dreidimensionale, bewegliche, verformbare und gegebenenfalls lebende Körper, wie z.B. menschliche Körper, reproduzierbar zu positionieren oder zu lagern oder Lage- oder Haltungsveränderungen solcher Körper möglichst präzise feststellen zu können.

Im medizin-technischen Bereich bestehen solche Notwendigkeiten insbesondere bei der perkutanen Strahlentherapie, bei der Patienten über einen Zeitraum von oftmals mehreren Wochen täglich bestrahlt werden müssen.

Menschliche Körper sind keine starren Gebilde, die ohne weiteres reproduzierbar ausrichtbar und lagerbar sind. Vielmehr ist der menschliche Körper beweglich, so daß es nicht unproblematisch ist, einen menschlichen Körper so exakt einzustellen, daß z.B. bei zeitlich aufeinander abfolgenden Bestrahlungssitzungen sichergestellt ist, daß das Isozentrum der Anordnung der Bestrahlungsfelder immer mit dem selben Raumpunkt im Patienten zusammenfällt, der in der dreidimensionalen Bestrahlungsplanung festgelegt worden ist.

In der Vergangenheit wurden unterschiedliche Methoden erprobt, um eine möglichst exakte reproduzierbare Patientenlagerung zu sichern. So wurde in den 50er Jahren eine "Nagelbrettmethode" angewandt, um einen einmal unter einem Bestrahlungsgerät gelagerten Patientenkörper immer wieder in dieselbe Position zu bringen, um möglichst reproduzierbare Bestrahlungsergebnisse sicherzustellen.

Später wurde versucht, mittels Laser-Abtastung einen Patientenkörper oberflächlich zweidimensional oder dreidimensional abzutasten, was aber nur zu begrenzten Erfolgen führte.

In jüngerer Zeit wurde das bei der Bestrahlung zu erfassende Zielvolumen klinisch mit Hilfe von CT-, MR- und/oder anderen bildgebenden Verfahren festgelegt. Anschließend wurde auf Basis der so ermittelten Daten eine physikalische Bestrahlungsplanung durchgeführt, wobei aber immer bedacht werden mußte, daß die räumliche Auflösung der computergestützten Planung maximal nur dem Auflösungsvermögen des zugrundeliegenden bildgebenden Verfahrens entsprechen kann. Die konventionellen Verfahren der Lagerung für die einzelnen Therapiesitzungen machen diese Genauigkeit aber wieder zunichte.

Bei den heute eingesetzten Bestrahlungsapplikationen werden üblicherweise isozentrische Bestrahlungstechniken eingesetzt, bei denen das Zentrum des Zielvolumens mit dem Zentrum der Bestrahlungsanlage zur Deckung gebracht werden muß. In der Praxis erweist sich dies als schwierig, da das Volumen in der Mehrzahl der Fälle nicht an der Oberfläche liegt und nur mit Hilfe von radiographischen Methoden eindeutig lokalisiert werden kann.

Die bisherigen Lagerungstechniken stützen sich daher oftmals auf raumfeste Orientierungslaser, die das Isozentrum vorgeben und deren Auftretungspunkt auf der Haut mit Hautmarkierungen zur Deckung gebracht werden muß. Zusätzlich werden in vielen Fällen Formkissen und Masken eingesetzt, die die Bestrahlungsregion großräumig mechanisch fixieren sollen.

Diese am weitesten verbreitete Lagerungstechnik bringt aber oftmals geometrische Fehler mit sich, die bei einer Bestrahlung im Bereich des Beckens ohne Lagerungskissen, z.B. als maximaler Fehler von 2,5 cm angegeben werden, bei einem mittleren Fehler (90 % der ermittelten Patienten) von 1 cm.

Bei der Bestrahlung im Kopf- bzw. Halsbereich werden in der Regel die schon erwähnten Masken eingesetzt, die ermittelten Abweichungen sind in diesem Falle um Faktor 2 kleiner.

Die Hauptfehlerquellen bei der Lagerung sind unterschiedliche Gerätegeometrien von Simulator und Bestrahlungsgerät innerhalb einer Abteilung, die auf der Patientenhaut angebrachten Hautmarkierungen an sich, weil die Haut gegenüber dem Skelett und den inneren Organen leicht verschoben werden kann, sowie die relativ breiten Markierungsstriche, da sie mit der Zeit undeut-lich werden und auch die Gefahr besteht, daß sie auf dem Patientenkörper falsch nachgezeichnet werden. Weitere Fehlerquellen sind Figuränderungen des Patienten, wobei zu bedenken ist, daß sich das Körpergewicht innerhalb einer vergleichsweise langen Behandlungszeit zum Teil dramatisch ändern kann, was sich wiederum auf die Form der Hautkontur und damit auf die Lage der Markierungen auswirkt. Als weitere Fehlerquelle sind auch die Eigenbewegungen eines menschlichen Körpers , z.B. bei der Atmung, mit in Betracht zu ziehen.

Aus US-A-5 446 548 ist ein Verfahren zur reproduzierbaren Positions- oder Haltungserkennung oder Lagerung von lebenden Körpern bekannt, bei welchem Oberflächenpunkte eines Patienten mittels zweier Kameras erfaßt und abgespeichert werden. Auf den Patientenkörper müssen also vor Durchführung des Verfahrens gesonderte Diskretemarkierungen angebracht werden. Die 3-dimensionalen Oberflächenpunkte werden - nach Neulagerung des Patienten - mit den ursprünglichen verglichen und die Neupositionierung unter Minimalisierung der Lagedifferenz durchgeführt.

Aus DE 34 36 444 A1 ist ein Verfahren sowie eine Einrichtung zum 3-dimensionalproduzierbaren Positionieren eines Patienten bekannt, wobei wenigstens zwei Bezugsbilder des Patienten in zwei verschiedenen Bildebenen bei einer ersten exakten Positionierung des Patienten aufgenommen und bei einer späteren zu reproduzierenden Positionierung dieses Patienten mit entsprechenden aktuellen Bildern verglichen werden. Die Positionierung des Patienten wird dabei so lange verändert, bis die jeweiligen Bezugsbilder mit den entsprechenden aktuellen Bildern, welche bevorzugt fortlaufend erzeugt werden, übereinstimmen.

### Aufgabenstellung/Lösung:

Der Erfindung liegt die Aufgabe zugrunde, ein Verfahren zur reproduzierbaren Positions- oder Halterungserkennung oder Lagerung von dreidimensionalen Körpern oder Körperteilen anzugeben, das schnell und genau durchführbar ist, aus dem sich klare Anleitungen für eine Lagekorrektur ableiten lassen, das unabhängig von diskreten Markierungen arbeitet und ohne eine relative Scanbewegung zwischen Patientenkörper oder Detektoranordnung auskommt und insbesondere für Objekte einsetzbar sein soll, deren Oberflächen diffus reflektierend sind. Zusätzlich soll das Verfahren auch eine quantitative vergleichende Analyse von Bewegungen und Bewegungsabläufen ermöglichen.

Diese Aufgabe wird durch die Merkmalskombination des Patentanspruchs 1 gelöst. Vorteilhafte Weiterbildungen des Verfahrens ergeben sich aus den Unteransprüchen 2 - 21.

Als Kern des erfindungsgemäßen Verfahrensablaufs wird es angesehen, einen Körper auf einer ersten Unterlage in einer Ausgangsposition zu positionieren, sodann eine erste Untersuchungsmethode unter Beibehaltung der Ausgangsposition zum optischen Erfassen der Oberfläche des Körpers mittels mindestens eines 3D-Sensorsystems durchzuführen, bei welcher Oberflächenpunkte des Körpers bezüglich ihrer dreidimensionalen Raumlage erfaßt und als Satz dreidimensionaler Koordinaten in einem Rechnersystem abgespeichert werden. Der Körper wird sodann erneut positioniert, und zwar entweder wieder auf der ersten oder einer anderen Oberfläche, beispielsweise im medizinischen Bereich auf der Lagerfläche eines Bestrahlungsgerätes. Die erste Untersuchungsmethode wird erneut angewandt oder durchgeführt, durch welche wiederum Oberflächenpunkte bezüglich ihrer Raumlage optisch erfaßt und als weiterer Satz dreidimensionaler Koordinaten abgespeichert werden. Anschließend werden die mindestens zwei abgespeicherten Oberflächendatensätze aus den beiden Durchführungen der Untersuchungsmethoden verglichen, aus dem Vergleich wird eine Lägeabweichungsinformation gewonnen, die dann dazu herangezogen werden kann, die Lage des Patientenkörpers zu korrigieren oder Bewegungsabläufe und/oder Fehlhaltungen des Körpers zu erkennen.

Erfindungsgemäß wird bei der Durchführung des Verfahrens das Meßprinzip der phasenmessenden Triangulation eingesetzt.

Zur Durchführung des Verfahrens wird der Patientenkörper aus mehreren Richtungen gleichzeitig oder wechselseitig abgetastet und die resultierenden Oberflächen aller einzelnen Sensoren durch einheitliche Kalibrierung an einem gemeinsamen Punkt zu einer vollständigen Oberfläche überlagert. Der Kalibrierungspunkt wird dabei zum Koordinatenursprung, die Oberfläche wird in Relativkoordinaten dazu angegeben und kann so in einem Rechner weiterverarbeitet werden.

Die Wellenlänge des verwendeten Meßlichtes kann im sichtbaren Bereich angesiedelt sein. Liegt sie aber im IR-Bereich, hätte dies den Vorteil, daß auch dunklere Oberflächen mehr Meßlicht reflektieren, eventuell vorhandene Haare auf der Oberfläche stören dann weniger, und ein menschlicher Körper wird bei einer Vermessung einer Region im Augenbereich weniger stark irritiert.

Das Verfahren sieht in einer vorteilhaften Weiterbildung auch vor, daß neben der äußeren Struktur eines Körpers auch dessen innere Strukturen erfaßt werden. Anhand der Bilddaten aus dem bildgebenden Verfahren für die inneren Strukturen, d.h. für das Volumen, kann ebenfalls die Oberfläche des Körpers in diesem Bereich aus den Volumendaten rekonstruiert werden und dient damit als Matritze für die optisch topometrierte. Die beiden Oberflächendatensätze werden in einem Rechner für die Bilddarstellung des Körpers zur Deckung gebracht. Die Lage der interessierenden inneren Strukturen, insbesondere ein Zielpunkt für eine Bestrahlung wird dann aus dem Volumendatensatz entnommen und allein in räumlicher Relation zur ermittelten Oberflächen dargestellt und weiterverarbeitet.

Wird ein scannendes Verfahren für die Bildgebung verwendet, z. B. die Computertomographie für die Erfassung der inneren Strukturen, so kann für eine möglichst simultane Oberflächentopometrie auch ein Lichtschnittverfahren benutzt werden.

In vorteilhafter Weise ist es auch möglich, Lageabweichungsinformationen zur Durchführung eines interaktiven Korrekturverfahrens zur Lagekorrektur des Körpers zu visualisieren. Dabei ist es möglich, die Lageabweichungsinformation auch zur Steuerung einer auf den Körper einwirkenden Lageabweichungskorrekturvorrichtung zu verwenden.

Wird die Lageabweichungsinformation visualisiert, so ist es im Rahmen des erfindungsgemäßen Verfahrens vorgesehen, den Körper auf oder über der Unterlage so lange zu bewegen, bis auf einer Anzeigevorrichtung die visualisierten Oberflächendastellungen weitgehend deckungsgleich übereinander liegen, wodurch die gewünschte Ausgangs- oder Sollposition sichergestellt ist.

Bei der Korrekturbewegung des Körpers, die automatisch oder manuell durchgeführt werden kann, werden vorteilhafterweise fortlaufend neue Oberflächendatensätze gewonnen und verarbeitet, d.h. mit bereits vorher abgespeicherten Oberflächendatensätze verglichen und daraus rechnerisch neue Lageabweichungsinformationen ermittelt.

Besonders leicht durchführbar wird das Lagekorrekturverfahren für das Betreuungspersonal dann, wenn die Oberflächendarstellungen, die aus zeitlich nacheinander durchgeführten Untersuchungsmethoden gewonnen werden, auf der Anzeigevorrichtung unterschiedliche Farben aufweisen. Der Oberflächendatensatz, der aus einem ersten Untersuchungsdurchgang gewonnen wurde, kann beispielsweise grün dargestellt werden, ein zweiter Oberflächendatensatz führt dann auf dem gleichen Bildschirm beispielsweise zu einer roten Abbildung des Körpers.

Stellt die grüne Körperabbildung auf der Anzeigevorrichtung die Sollage des Körpers dar, muß der Körper solange verschoben werden, bis die rote Darstellung, die aus immer wieder neugewonnenen Datensätzen ermittelt wird, unter der grünen Abbildung liegt.

Es ist auch möglich, nur die Abweichungen der Körperdarstellungen von der Ausgangsposition auf der Anzeigevorrichtung unterschiedlich einzufärben, d.h. Lageübereinstimmungen von gewissen Körperbereichen sind dann nicht mehr als unterschiedlich eingefärbt zu sehen, sondern nur noch abweichende Körperteile. Um das Verfahren noch komfortabler durchführen zu können, ist es auch möglich, Lageabweichungen quantitativ durch unterschiedliche Farbintensitäten darzustellen, so daß z.B. starke Abweichungen im Plusbereich dunkelrot sind und nur noch schwache Abweichungen im hellroten Bereich dargestellt werden. Dies gibt dem Betreuer des Verfahrens wertvolle Informationen, wie er die Lage des Körpers zu korrigieren hat.

Vorteilhaft kann es auch sein, wenn bezogen auf eine festgelegte Raumkoordinate Plusabweichungen von der Ausgangssollage farblich oder auf sonstige Weise optisch unterscheidbar von Minusabweichungen von der Ausgangssollage dargestellt werden. Mit anderen Worten kann z.B. ein Körperbereich, der bezogen auf die Sollage zu hoch liegt, grün dargestellt werden, liegt er hingegen zu niedrig, kann er rot dargestellt werden. Dadurch wird eine Lagekorrektur besonders einfach ermöglicht.

Zusammenfassend kann festgestellt werden, daß das Verfahren eine genaue und reproduzierbare Lokalisation von inneren Strukturen ohne kostspielige, zeitraubende oder möglicherweise dosisbelastende bildgebende Verfahren ermöglicht, sofern neben der ersten Untersuchungsmethode zum optischen Erfassen der Oberfläche des Körpers eine zweite Untersuchungsmethode eingesetzt wird, mit der innere Körperstrukturen erfaßt werden können. Dies gilt solange, wie die räumliche Korrelation zwischen innerer Struktur und äußerer Form einen bestimmten Toleranzwert nicht überschreitet.

Besonders hervorzuheben ist die Genauigkeit der Positionierung, die dem physikalischen Auflösungsvermögen der Topometrie entspricht. Bei einer angenommenen maximalen Meßfeldgröße von 80 x 80 cm erreicht die Positionsgenauigkeit 0,8 mm.

Es ist weiterhin mit Vorteil möglich, die genaue Lage des Körpers über einen längeren Zeitraum hinweg automatisch zu erfassen und während einer Bestrahlungssitzung auch zu protokollieren. Bei strahlentherapeutischer Behandlung ermöglicht dies die Bewertung, ob die geplanten Behandlungsvolumina ausreichend bemessen sind, was eine wichtige Aufgabe der Qualitätssicherung in diesem medizinischen Bereich darstellt.

Bei der strahlentherapeutischen Anwendung ergibt sich mit Vorteil, daß Lokalisation und Simulation unter Durchleuchtung, zeitaufwendige Standardprozeduren der klinischen Routine ohne Patientenbeteiligung virtuell am Planungsrechner durchgeführt werden können. Für strahlentherapeutische Anwendungen müssen Patienten nicht mehr auf der Haut markiert werden, was eine Entlastung der strapazierten Hautbereiche und eine psychologische Erleichterung für den Patienten bedeutet.

Wird das Verfahren für eine vergleichende dreidimensionale und quantitative Analyse von Bewegungssequenzen in Echtzeit verwendet, mithin außerhalb der Strahlentherapie angewandt, so läßt es sich für die Erkennung von Bewegungen und damit z.B. auch für die Identifikation von Objekten verwendeten Das System erlaubt dabei u.a. eine Analyse einer dreidimensionalen Form und der Dynamik, z.B. des Gesichts einer Person, und damit eine nichtinvasive schnelle und hochspezifische Identifikation durch Wiedererkennung. Überträgt man diese Erkenntnis auf den medizinischen Bereich, so kann durch Einsatz der vorliegenden Erfindung sichergestellt werden, daß Fehlbehandlungen von Patienten vermieden werden, was beispielsweise durch Laden falscher Patientendaten in einem Rechner geschehen kann. Da der Rechner automatisch den Patienten aufgrund des Oberflächendatensatzes erkennt, wird er die Annahme falscher Patientendaten verweigern und nachfolgende Fehlbehandlungen am Patienten nicht zur Durchführung freigeben.

## Patentansprüche

1. Verfahren zur reproduzierbaren Positions- oder Haltungserkennung oder Lagerung von dreidimensionalen, beweglichen und verformbaren Körpern oder Körperteilen, insbesondere lebenden Körpern, bezogen auf eine Unterlage und/oder einen über diesen definierten Raumpunkt oder ein Koordinatensystem mit folgenden Verfahrensschritten:
a) Positionierung des Körpers auf einer ersten Unterlage in einer Ausgangsposition,
b) Durchführung oder Anwendung einer ersten Untersuchungsmethode unter Beibehaltung der Ausgangsposition zum optischen Erfassen der Oberfläche des Körpers, wobei Oberflächenpunkte des Körpers mittels mindestens eines 3D-Sensorsystems bezüglich ihrer dreidimensionalen Raumlage erfaßt und als Satz dreidimensionaler Koordinaten abgespeichert werden,
c) erneute Positionierung des Körpers auf der ersten oder einer anderen Oberfläche, erneute Durchführung oder Anwendung der ersten Untersuchungsmethode, durch welche erneut Oberflächenpunkte bezüglich ihrer Raumlage optisch erfaßt und als weiterer Satz dreidimensionaler Koordinat en abgespeichert werden,
d) Vergleich der mindestens zwei abgespeicherten Oberflächendatensätze aus den beiden Durchführungen der Untersuchungsmethode(n) sowie
e) daraus Gewinnung einer Lageabweichungsinformation,
**gekennzeichnet durch**
f) den Einsatz einer phasenmessenden Triangulations-Topometrie einrichtung, wobei
g) die Körperoberfläche unmittelbar ohne Einsatz von diskreten Markierungen abge tastet wird.

2. Verfahren nach dem Ansprüch 1,
**dadurch gekennzeichnet, daß**
mindestens zwei 3D-Sensorsysteme, die den Körper aus unterschiedlichen Richtungen abtasten, verwendet werden.

3. Verfahren nach Anspruch 1 oder 2,
**dadurch gekennzeichnet, daß**
mit den Sensorsystemen der Körper gleichzeitig oder wechselseitig abgetastet wird.

4. Verfahren nach einem der vorhergehenden Ansprüche,
**dadurch gekennzeichnet, daß**
die Wellenlänge des eingesetzten Meßlichtes des 3D-Sensorsystems im IR-Bereich liegt.

5. Verfahren nach einem der vorhergehenden Ansprüche,
**dadurch gekennzeichnet, daß**
die Lageabweichungsinformation zur Durchführung eines interaktiven Korrekturverfahrens zur Lagekorrektur des Körpers visualisiert wird.

6. Verfahren nach einem der vorhergehenden Ansprüche,
**dadurch gekennzeichnet, daß**
die Lageabweichungsinformation zur Steuerung einer Lageabweichungskorrekturvorrichtung für den Körper verwendet wird.

7. Verfahren nach einem der vorhergehenden Ansprüche,
**dadurch gekennzeichnet, daß**
vorlaufend, gleichzeitig oder nachfolgend zur ersten Untersuchungsmethode eine weitere oder dritte Untersuchungsmethode (CT-, MR-, Ultraschall-, PET (Positron-Emissions-Tomografie)) zum Erfassen der Lage und/oder Definition innerer Bereiche und/oder Strukturen des Körpers angewandt oder durchgeführt wird.

8. Verfahren nach einem der vorhergehenden Ansprüche,
**dadurch gekennzeichnet, daß**
der Körper unter Beobachtung der Körperoberflächenvisualisierung auf oder über der Unterlage solange bewegt wird, bis auf einer Anzeigevorrichtung die visualisierten Oberflächendarstellungen weitgehend deckungsgleich übereinander liegen, womit die bei der ersten Messung vorliegende Ausgangsposition wieder hergestellt ist.

9. Verfahren nach einem der vorhergehenden Ansprüche,
**dadurch gekennzeichnet, daß**
bei der Korrekturbewegung des Körpers fortlaufend neue Oberflächendatensätze gewonnen und verarbeitet werden und daraus neue Lageabweichungsinformationen gewonnen werden.

10. Verfahren nach einem der vorhergehenden Ansprüche,
**dadurch gekennzeichnet, daß**
die Oberflächendarstellungen, die aus zeitlich nacheinander durchgeführten Untersuchungsmethoden gewonnen werden, auf der Anzeigevorrichtung unterschiedliche Farben aufweisen.

11. Verfahren nach einem der vorhergehenden Ansprüche,
**dadurch gekennzeichnet, daß**
Abweichungen von der Ausgangsposition bei der Vergleichsdarstellung auf der Anzeigevorrichtung unterschiedliche Farben haben.

12. Verfahren nach einem der vorhergehenden Ansprüche,
**dadurch gekennzeichnet, daß**
bezogen auf eine festgelegte Raumkoordinate Plusabweichungen von der Ausgangsposition farblich oder auf sonstige Weise optisch unterscheidbar von Minusabweichungen von der Ausgangsposition auf der Anzeigevorrichtung dargestellt sind.

13. Verfahren nach einem der vorhergehenden Ansprüche,
**dadurch gekennzeichnet, daß**
die Bewegung des Körpers auf oder über der Oberfläche zu seiner Lagekorrektur manuell erfolgt.

14. Verfahren nach einem der vorhergehenden Ansprüche,
**dadurch gekennzeichnet, daß**
die Bewegung des Körpers zu seiner Lagekorrektur automatisch erfolgt, wobei die optisch gewonnenen Datensätze ausgewertet und Abweichungsdatensätze in eine die Lage des Körpers beeinflussende Lagesteuerungsvorrichtung eingegeben werden und die Lagesteuerungsvorrichtung die Lage des Körpers solange korrigiert, bis die Abweichungsdatensätze minimale Sollwerte einnehmen.

15. Verfahren nach einem der vorhergehenden Ansprüche,
**dadurch gekennzeichnet, daß**
aus den abgespeicherten Ergebnissen der weiteren Untersuchungsmethode und deren Korrelation mit den Ergebnissen der nach gegebenen-falls erneuter Positionierung des Körpers durchgeführten ersten Untersuchungsmethode nach gegebenenfalls erneuter Positionierung des Körpers die genaue Lage innerer Strukturen oder Bereiche des Körpers festgelegt werden kann.

16. Verfahren nach einem der vorhergehenden Ansprüche,
**dadurch gekennzeichnet, daß**
es zur reproduzierbaren Lagerung eines insbesondere menschlichen Patientenkörpers in diagnostischen oder therapeutischen Vorrichtungen angewandt wird.

17. Verfahren nach einem der vorhergehenden Ansprüche,
**dadurch gekennzeichnet, daß**
die zweite Untersuchungsmethode zum Erfassen der Lage und Definition innerer Bereiche des Körpers, insbesondere eine Röntgen-, CT-, MR-, Ultraschall-, PET- oder MEG-Methode ist.

18. Verfahren nach einem der vorhergehenden Ansprüche,
**dadurch gekennzeichnet, daß**
die erste Unterlage ein Patiententisch eines Diagnosegerätes ist und der Patient bei seiner erneuten Positionierung auf einer anderen Oberfläche positioniert wird, die ein Patiententisch einer therapeutischen Einrichtung ist, insbesondere Teil einer Bestrahlungsvorrichtung oder eines Operationstisches.

19. Verfahren nach einem der vorhergehenden Ansprüche,
**dadurch gekennzeichnet, daß**
zusätzlich zu dem im Rahmen der zweiten Untersuchungsmethode gewonnenen Oberflächeninformationsdatensatz betreffend den Körper ein weiterer Oberflächeninformationsdatensatz aus der ersten Untersuchungsmethode gewonnen wird, der mit dem Datensatz aus der zweiten Untersuchungsmethode kombiniert wird.

20. Verfahren nach einem der vorhergehenden Ansprüche,
**gekennzeichnet durch**
eine automatische Lageerfassung und Protokollierung des Körpers, insbesondere über einen Behandlungszeitraum hinweg.

21. Verfahren nach einem der vorhergehenden Ansprüche,
**dadurch gekennzeichnet, daß**
bei erneuter Lagerung des Körpers durch das Verfahren gewonnene Informationen betreffend den Körper diese Informationen mit bereits vorher erfaßten und abgespeicherten Informationen verglichen werden und daraus Patientenidentifizierungsinformation gewonnen wird.

## Claims

1. Method for the reproducible position- or attitude-detection or location of three-dimensional, displaceable and deformable bodies or body parts, more especially living bodies, relative to a base and/or a spatial point, defined via said base, or a co-ordinate system, said method having the following method steps:
a) positioning the body on a first base in an initial position,
b) accomplishing or using a first investigation method by maintaining the initial position for the optical detection of the surface of the body, surface points of the body being detected in respect of their three-dimensional spatial position by means of at least one 3D sensor system and stored as a set of three-dimensional co-ordinates,
c) fresh positioning of the body on the first or another surface, fresh accomplishment or use of the first investigation method, whereby surface points are optically detected afresh in respect of their spatial position and are stored as additional as an additional set of three-dimensional co-ordinates,
d) comparison of the at least two stored sets of surface data from the two accomplishments of the investigation method(s) and
e) obtaining therefrom information relating to position deviations,
**characterised by**
f) the use of a phase-measuring triangulation topometry apparatus,
g) the body surface being scanned directly without the use of discrete markers.

2. Method according to claim 1, **characterised in that** at least two 3D sensor systems are employed, which scan the body from different directions.

3. Method according to claim 1 or 2, **characterised in that** the body is scanned simultaneously or alternately with the sensor systems.

4. Method according to one of the preceding claims, **characterised in that** the wavelength of the measuring light of the 3D sensor system used lies within the IR range.

5. Method according to one of the preceding claims, **characterised in that** information relating to position deviations is visualised for accomplishing an interactive correction method for the positional correction of the body.

6. Method according to one of the preceding claims, **characterised in that** the information relating to position deviations is used to control a position deviation correction apparatus for the body.

7. Method according to one of the preceding claims, **characterised in that**, prior to, simultaneously with or subsequent to the first investigation method, an additional or third investigation method (CT method, MR method, an additional or third investigation method (CT method, MR method, ultrasound method, PET (positron emission tomography)) is applied or accomplished for detecting the position and/or definition of internal regions and/or structures of the body.

8. Method according to one of the preceding claims, **characterised in that** the body is displaced on or over the base, under observation of the body surface visualisation, until the visualised surface illustrations lie above one another in a largely congruent manner on a display apparatus, whereby the initial position, existing at the time of the first measurement, is restored.

9. Method according to one of the preceding claims, **characterised in that**, during the correction displacement of the body, new sets of surface data are continuously obtained and processed, and new information relating to position deviations is obtained therefrom.

10. Method according to one of the preceding claims, **characterised in that** the surface illustrations, which are obtained from investigation methods accomplished one after the other with respect to time, exhibit different colours on the display apparatus.

11. Method according to one of the preceding claims, **characterised in that** deviations from the initial position have different colours in the comparative illustration on the display apparatus.

12. Method according to one of the preceding claims, **characterised in that**, relative to a stipulated spatial co-ordinate, plus deviations from the initial position are illustrated in colour or in some other manner optically distinguishable from minus deviations from the initial position on the display apparatus.

13. Method according to one of the preceding claims, **characterised in that** the displacement of the body on or over the surface towards its position correction is carried out manually.

14. Method according to one of the preceding claims, **characterised in that** the displacement of the body towards its position correction is effected automatically, the sets of data obtained optically being evaluated and sets of deviation data being input into a position control apparatus, which influences the position of the body, and the position control apparatus correcting the position of the body until the sets of deviation data assume minimum desired values.

15. Method according to one of the preceding claims, **characterised in that**, from the stored results of the additional investigation method and the correlation thereof with the results of the first investigation method, accomplished after a possibly fresh positioning of the body, the exact position of internal structures or regions of the body can be ascertained after a possibly fresh positioning of the body.

16. Method according to one of the preceding claims, **characterised in that** it is applied to the reproducible location of a more especially human patient's body in diagnostic or therapeutic apparatuses.

17. Method according to one of the preceding claims, **characterised in that** the second investigation method for detecting the position and definition of internal regions of the body is, more especially, an X-ray, CT, MR, ultrasound, PET or MEG method.

18. Method according to one of the preceding claims, **characterised in that** the first base is a patient table of a diagnostic apparatus, and the patient is positioned on another surface for his fresh positioning, which surface is a patient table of a therapeutic apparatus, more especially a part of an irradiation apparatus or an operating table.

19. Method according to one of the preceding claims, **characterised in that**, in addition to the set of surface information data relating to the body, obtained within the scope of the second investigation method, an additional set of surface information data is obtained from the first investigation method, which is combined with the set of data from the second investigation method.

20. Method according to one of the preceding claims, **characterised by** an automatic position detection and recording of the body, more especially over a treatment period.

21. Method according to one of the preceding claims, **characterised in that** information relating to the body is obtained by the method for a fresh location of the body, said information is compared with information already previously detected and stored, and patient identification information is obtained therefrom.

## Revendications

1. Procédé pour l'identification ou le stockage reproductible de la position ou de la posture de corps ou parties de corps en trois dimensions mobiles et déformables, en particulier de corps vivants, relativement à une surface d'appui et/ou à un point dans l'espace défini au moyen de cette surface d'appui, ou à un système de coordonnées, comprenant les étapes de procédé suivantes :
a) positionnement du corps sur une première surface d'appui dans une position initiale,
b) exécution ou application d'une première méthode d'analyse, en conservant la position initiale, pour la détection optique de la surface du corps, sachant que la position dans l'espace tridimensionnel de points de la surface du corps est détectée au moyen d'un système de détection à 3 dimensions et est mémorisée sous la forme d'un jeu de coordonnées en trois dimensions,
c) nouveau positionnement du corps sur la première surface ou sur une autre surface, nouvelle exécution ou application de la première méthode d'analyse, par laquelle, à nouveau, la position dans l'espace de points de la surface est détectée et mémorisée sous la forme d'un autre jeu de coordonnées en trois dimensions,
d) comparaison des au moins deux jeux de données de surface mémorisés provenant des deux exécutions de la ou des méthodes d'analyse,
e) et obtention, à partir de cette comparaison, d'une information d'écart de position,
**caractérisé par**
f) l'utilisation d'un appareil de topométrie par triangulation à mesure de phase,
g) sachant que la surface du corps est directement balayée, sans utilisation de repères discrets.

2. Procédé selon la revendication 1, **caractérisé en ce qu'**on utilise au moins deux systèmes de détection à 3 dimensions, qui balayent le corps à partir de directions différentes.

3. Procédé selon la revendication 1 ou 2, **caractérisé en ce que** le corps est balayé simultanément ou alternativement par les systèmes de détection.

4. Procédé selon l'une des revendications précédentes, **caractérisé en ce que** la longueur d'onde de la lumière de mesure utilisée par le système de détection à 3 dimensions se situe dans la plage infrarouge.

5. Procédé selon l'une des revendications précédentes, **caractérisé en ce que** l'information d'écart de position est visualisée afin de mettre en oeuvre une procédure de correction interactive pour corriger la position du corps.

6. Procédé selon l'une des revendications précédentes, **caractérisé en ce que** l'information d'écart de position est utilisée pour commander un dispositif de correction d'écart de position pour le corps.

7. Procédé selon l'une des revendications précédentes, **caractérisé en ce que**, avant, après ou en même temps que la première méthode d'analyse, on applique ou exécute une autre ou troisième méthode d'analyse (tomodensitométrie, tomographie par résonance magnétique, échographie, TEP (tomographie d'émission par positons)) pour définir et/ou détecter la position de structures et/ou de régions internes du corps.

8. Procédé selon l'une des revendications précédentes, **caractérisé en ce que** le corps est, tout en observant la visualisation de la surface du corps, déplacé sur ou au moyen de la surface d'appui jusqu'à ce que les représentations de la surface visualisées sur un dispositif d'affichage soient superposées de manière essentiellement coïncidente, rétablissant ainsi la position initiale présente lors de la première mesure.

9. Procédé selon l'une des revendications précédentes, **caractérisé en ce que**, lors du mouvement de correction du corps, on obtient et on traite en continu de nouveaux jeux de données de surface et on en tire de nouvelles informations d'écart de position.

10. Procédé selon l'une des revendications précédentes, **caractérisé en ce que** les représentations de surface qui ont été obtenues à partir de méthode d'analyse exécutées successivement dans le temps présentent des couleurs différentes sur le dispositif d'affichage.

11. Procédé selon l'une des revendications précédentes, **caractérisé en ce que** des écarts par rapport à la position initiale possèdent des couleurs différentes dans la représentation comparative sur le dispositif d'affichage.

12. Procédé selon l'une des revendications précédentes, **caractérisé en ce que**, relativement à une coordonnée spatiale définie, des écarts positifs par rapport à la position initiale sont représentés sur le dispositif d'affichage, par une coloration ou d'une autre manière, d'une manière optiquement distinguable d'écarts négatifs par rapport à la position initiale.

13. Procédé selon l'une des revendications précédentes, **caractérisé en ce que** le déplacement du corps sur ou au moyen de la surface pour corriger sa position s'effectue de façon manuelle.

14. Procédé selon l'une des revendications précédentes, **caractérisé en ce que** le déplacement du corps pour corriger sa position s'effectue de façon automatique, sachant que les jeux de données obtenus optiquement sont évalués et que des jeux de données d'écart sont introduits dans un dispositif de commande de position agissant sur la position du corps, et le dispositif de commande de position corrige la position du corps jusqu'à ce que les jeux de données d'écart prennent des valeurs de consigne minimales.

15. Procédé selon l'une des revendications précédentes, **caractérisé en ce que**, à partir des résultats mémorisés de l'autre méthode d'analyse et de leur corrélation avec les résultats de la première méthode d'analyse exécutée après un éventuel nouveau positionnement du corps, on peut définir, après un éventuel nouveau positionnement du corps, la position précise de régions ou structures internes du corps.

16. Procédé selon l'une des revendications précédentes, **caractérisé en ce qu'**il est appliqué pour le stockage reproductible d'un corps de patient, notamment humain, dans des dispositifs diagnostiques ou thérapeutiques.

17. Procédé selon l'une des revendications précédentes, **caractérisé en ce que** la deuxième méthode d'analyse de détection de position et de définition de régions internes du corps est notamment une méthode à rayons X, de tomodensitométrie, à résonance magnétique, à ultrasons, de tomographie d'émission par positons ou à magnétoencéphalogramme.

18. Procédé selon l'une des revendications précédentes, **caractérisé en ce que** la première surface d'appui est une table de patient d'un appareil de diagnostic et le patient est, lors de son nouveau positionnement, positionné sur une autre surface qui est une table de patient d'un appareil thérapeutique, notamment qui fait partie d'un dispositif d'irradiation ou d'une table d'opération.

19. Procédé selon l'une des revendications précédentes, **caractérisé en ce que**, en plus du jeu de données d'information de surface concernant le corps qui est obtenu dans le cadre de la deuxième méthode d'analyse, on obtient un autre jeu de données d'information de surface à partir de la première méthode d'analyse, qui est combiné avec le jeu de données issu de la deuxième méthode d'analyse.

20. Procédé selon l'une des revendications précédentes, **caractérisé par** une détection et une consignation automatiques de la position du corps, en particulier sur une période de traitement.

21. Procédé selon l'une des revendications précédentes, **caractérisé en ce que**, lors du nouveau stockage du corps au moyen d'informations concernant le corps qui sont obtenues par le procédé, ces informations sont comparées aux informations déjà détectées et mémorisées, et on en tire une information d'identification du patient.
